# EUROPEAN PATENT SPECIFICATION

(11) **EP 1 744 725 B1**
(45) Date of publication and mention of the grant of the patent: **29.07.2009**
(21) Application number: 05721579.0
(22) Date of filing: 22.03.2005
(51) Int. Cl.: A61K 8/34, A61K 8/60, A61Q 19/00

(54) **A SKIN CARE AND COSMETIC PREPARATION CONTAINING AN INOSITOL DERIVATIVE**
HAUTPFLEGE- UND KOSMETISCHES PRÄPART MIT EINEM INOSITOL-DERIVAT
UNE PRÉPARATION COSMÉTIQUE ET DERMATOLOGIQUE CONTENANT UN DÉRIVÉ INOSITOL

(30) Priority: 25.03.2004 JP 2004089591
(43) Date of publication of application: 24.01.2007
(73) Proprietor: Showa Denko K.K., Tokyo 105-8518 (JP)
(72) Inventor: AOKI, Hirobumi;, , Oonodai, Midori-ku, Chiba-shi, Chiba 2 (JP); KAMACHI, Motoaki;, Kawasaki-shi, Kanagawa 2100867; (JP)
(74) Representative: Strehl Schübel-Hopf & Partner
(86) International application number: PCT/JP2005/005652
(87) International publication number: WO 2005/092285

(56) References cited:
- FR-A- 2 748 031
- JP-A- 63 196 596
- US-A1- 2003 068 297
- US-B1- 6 417 339
- PATENT ABSTRACTS OF JAPAN vol. 012, no. 479 (C-552), 14 December 1988 (1988-12-14) & JP 63 196596 A (KURITA WATER IND LTD; others: 01), 15 August 1988 (1988-08-15)

## Description

### Technical Field

The present invention relates to the use of an external preparation for skin that contains an inositol derivative in which an inositol and a saccharide are combined. More particularly, the invention relates to the use of an external preparation for skin and the use of a cosmetic that have the effects of keeping the skin healthy.

### BACKGROUND OF THE INVENTION

Inositol is a constituent of phytin, and the phytin is known to be found in rice bran in a high proportion of about 10%. The rice bran extract contains, in addition to the inositol, fat and oil, vitamin B and minerals. Therefore, the rice bran extract has been used in skin cosmetics for providing oil, moisture and nutrient contents to the skin, for improving moist feeling and moisture retention, and for preventing elastic decrease.

It is known that higher levels of these effects can be achieved by cosmetics that contain polysaccharides extracted from rice bran as active ingredients, or that contain water-soluble antioxidants from the rice bran (for example, see Patent Documents 1 and 2).

Since the rice bran extract has a distinctive odor, there are cosmetics containing the odorless inositol that is selectively extracted from the rice bran or chemically synthesized (for example, see Patent Document 3).

Moreover, cosmetics are known that contain an inositol which is sugar alcohol for preventing stickiness of the cosmetics (for example, see Patent Document 4).

Although the inositols are synthesized in the human body, the inositol requirement cannot be reached by the synthesis; therefore, the compounds are classified as a member of the vitamins. The inositols reportedly have physiological activities to prevent cirrhosis hepatic, fatty liver and hardened arteries and also to promote growth. Known applications include cosmetics and drink additives (for example, see Patent Document 5).

On the other hand, in recent years various saccharide-modified compounds have become producible. For the inositols as well, they are converted to glucooligosaccharides for use as biochemical reagents and bacteria growth accelerating substances (for example, see Patent Document 6).
[Patent Document 1] JP-A-S64-66106
[Patent Document 2] JP-A-H02-53705
[Patent Document 3] JP-A-H10-114641
[Patent Document 4] JP-A-2000-161015
[Patent Document 5] JP-A-H07-330643
[Patent Document 6] JP-A-S63-196596

### DISCLOSURE OF THE INVENTION

### [Problems to be solved by the invention]

Although the rice bran is useful as an inositol source, its distinctive odor causes a problem that an external preparation for skin containing the rice bran has the distinctive odor. Addition of aroma chemicals has been attempted for masking the odor but has resulted in problems that the skin will be adversely affected by stimulation of the aroma chemicals and the odor of the aroma chemicals will be disagreeable to some individuals.

Furthermore, the rice bran extract has another problem that an insoluble ingredient is often precipitated in the skin external preparation and the user feels grains during application to the skin. The pure inositol extract and chemically synthesized inositols have reduced precipitation of insoluble ingredients, but are not always useful as an active ingredient for use in the skin external preparations in view of adhesion and compatibility with lipid-rich skin surface. As described above, the use of the rice bran extract and chemical compounds in the skin external preparations only results in unsatisfactory effects.

The present invention has an object of solving the aforesaid problems and defects by providing an external preparation for skin and a cosmetic that contain an inositol derivative. More specifically, it is an object of the invention to provide an external preparation for skin and a cosmetic that contain an inositol combined with a saccharide.

### [Means for solving the problems]

An earnest study for solving the abovementioned problems has resulted in the following findings: A derivative in which an inositol is modified with a saccharide shows excellent solubility so that precipitation of an insoluble ingredient can be suppressed when an external preparation for skin contains the derivative. Further, the skin external preparation exhibits greatly improved adhesion and compatibility with the skin, leading to remarkably enhanced skin's moist feeling and texture. The present invention has been completed based on these findings.

Namely, the present invention concerns to the following.
(1) The use of an external preparation for skin containing an inositol derivative in which an inositol is combined with a saccharide.
(2) The use of the external preparation for skin as described in (1), wherein the saccharide is a monosaccharide and/or an oligosaccharide.
(3) The use of the external preparation for skin as described in (2), wherein the monosaccharide and/or the oligosaccharide contains glucose as a constitutional unit.
(4) The use of the external preparation for skin as described in any of (1) to (3), wherein the inositol of the inositol derivative is myo-inositol.
(5) The use of the external preparation for skin as described in any of (1) to (4), wherein the inositol derivative in which the inositol is combined with the saccharide constitutes 0.01 to 50% by mass of the preparation.
(6) The use of a cosmetic containing the external preparation for skin as described in any of (1) to (5).

### BEST MODE FOR CARRYING OUT THE INVENTION

The inositol derivative used in the present invention is a compound resulting from combination of an inositol with a saccharide.

There is particularly no limitation on the saccharides for use in the invention. Exemplary saccharides include mannitol, sorbitol, xylitol, maltitol, erythritol, pentaerythritol, glucose, sucrose, fructose, lactose, maltose, xylose, trehalose, α-cyclodextrin, β-cyclodextrin and γ-cyclodextrin.

Of these saccharides, monosaccharides, oligosaccharides and mixtures thereof are preferable. In particular, glucose and/or oligosaccharides containing glucose as a constitutional unit are preferred.

The inositol used in the present invention is a cyclic hexahydric alcohol represented by C₆H₆(OH)₆ and has nine stereoisomers. The invention may employ any of the isomers. Of the inositol isomers, naturally occurring myo-inositol is particularly preferred. The inositol for use in the invention may be extracted from the rice bran or be synthesized by chemical synthesis methods or fermentation.

The inositol derivative is a compound in which the saccharide such as monosaccharide or oligosaccharide is combined to the hydroxyl group of the inositol. The saccharide may be combined to one or more of the six hydroxyl groups of the inositol, and is generally combined to one hydroxyl group. Further, the inositol derivative used in the invention may be such that a plurality of inositols are combined to one saccharide.

The inositol derivative may be synthesized by known methods without limitation. For example, the inositol may be reacted with cyclodextrin which is an oligosaccharide, in the presence of cyclodextrin glucanotransferase to afford a saccharide or oligosaccharide with a combined inositol residue (JP-A-S63-196596). Further, a glucosylated inositol derivative may be obtained by using glucosyl phosphite as a saccharide donor (JP-A-H06-298783).

The inositol derivative from combination of the inositol and saccharide may be compounded in a skin external preparation as follows: the solid, powdered or semisolid inositol derivative is admixed to a skin external preparation; an aqueous solution of the derivative is admixed to a skin external preparation, the derivative in the form of solution in an alcohol or other appropriate solvent is admixed to a skin external preparation, and the derivative is mixed with or added to a skin external preparation by known methods. Methods for the production of the skin external preparations are not limited to the aforementioned.

The inositol derivative for use in the invention may be isolated as white powder. An aqueous solution of the derivative obtained during the production may also be compounded in the external preparation for skin.

The inositol derivative in which the inositol is combined with the saccharide may be used in amounts that are not particularly limited as long as appropriate for the skin external preparation or cosmetic. Preferably, the derivative is used in amounts from 0.01 to 50% by mass, and more preferably from 0.1 to 10% by mass. When the amount of the inositol derivative is within the above range, the resultant skin external preparation provides comfortable feeling such as sufficient skin moisture retention and has appropriate viscosity. Accordingly, the skin external preparation can function satisfactorily.

The skin external preparations referred to in the invention are not particularly limited, and include cosmetics, cleansing agents, bath agents and soaps that are used directly on the skin. The cosmetics are a particularly preferred application.

The skin external preparations in the broad sense refer to any kinds of preparations that can be used in contact with skin regardless of user's gender and age. Examples thereof include skin milks, skin creams, foundation creams, massage creams, cleansing creams, shaving creams, cleansing foams, skin lotions, lotions, packs, lipsticks, rouges, eye shadows, manicures, soaps, body shampoos, hand soaps, shampoos, conditioners, hair tonics, treatment conditioners, hair creams, hair sprays, hair growth tonics, baldness remedies, hairdyes, styling spritz, depilatories, toothpastes, mouthwashes, permanent wave agents, ointments and bath agents.

The state of the skin external application may be solid, semisolid, liquid and gas. The form of the skin external preparation may be, although not particularly limited to, powder, granules, tablets, gels and foams.

In addition to the saccharide-combined inositol derivative, the skin external preparation of this invention may optionally contain an ingredient commonly used in skin external preparations without adversely affecting the effects of the invention.

Examples of such ingredients include:
hydrocarbons such as ozokerite, α-olefin oligomers, light isoparaffin, light liquid isoparaffin, squalene, squalane, synthetic squalane, vegetable squalane, ceresin, paraffin, polyethylene powder, polybutene, microcrystalline wax, liquid isoparaffin, liquid paraffin, mineral oil and vaseline;
natural waxes such as jojoba oil, carnauba wax, candelilla wax, rice bran wax, shellac, lanolin, mink oil wax, whale wax, sugarcane wax, sperm oil, beeswax and montan wax;
natural fats and oils such as avocado oil, almond oil, olive oil, extra virgin olive oil, sesame oil, rice bran oil, rice oil, rice germ oil, corn germ oil, safflower oil, soybean oil, corn oil, rapeseed oil, persic oil, palm kernel oil, palm oil, castor oil, sunflower oil, high oleic sunflower oil, grape seed oil, cotton seed oil, coconut oil, hydrogenated coconut oil, beef tallow, hardened oil, horse oil, mink oil, egg yolk oil, egg yolk fatty oil, rose hip oil, kukui nut oil, evening primrose oil, wheat germ oil, peanut oil, camellia oil, sasanqua oil, cacao butter, Japanese wax, beef bone fat, neatsfoot oil, lard, horse fat, mutton tallow, shea butter, macadamia nut oil and meadowfoam oil;
fatty acids such as lauric acid, myristic acid, palmitic acid, stearic acid, behenic acid, oleic acid, linolic acid, linolenic acid, γ-linolenic acid, isostearic acid, 12-hydroxystearic acid, undecylenic acid and coconut fatty acid;
higher alcohols such as isostearyl alcohol, octyldodecanol, hexyldecanol, cholesterol, phytosterol, lauryl alcohol, myristyl alcohol, cetanol, stearyl alcohol, oleyl alcohol, behenyl alcohol and cetostearyl alcohol;
alkyl glyceryl ethers such as batyl alcohol, chimyl alcohol, selachyl alcohol and isostearyl glyceryl ether;
esters such as isopropyl myristate, butyl myristate, isopropyl palmitate, ethyl stearate, butyl stearate, ethyl oleate, ethyl linoleate, isopropyl linoleate, cetyl caprylate, hexyl laurate, isooctyl myristate, decyl myristate, myristyl myristate, cetyl myristate, octadecyl myristate, cetyl palmitate, stearyl stearate, decyl oleate, oleyl oleate, cetyl ricinoleate, isostearyl laurate, isotridecyl myristate, isocetyl myristate, isostearyl myristate, octyldodecyl myristate, 2-ethylhexyl palmitate, isocetyl palmitate, isostearyl palmitate, 2-ethylhexyl stearate, isocetyl stearate, isodecyl oleate, octyldodecyl oleate, octyldodecyl ricinoleate, ethyl isostearate, isopropyl isostearate, cetyl 2-ethylhexanoate, cetostearyl 2-ethylhexanoate, stearyl 2-ethylhexanoate, hexyl isostearate, ethylene glycol dioctanoate, ethylene glycol dioleate, propylene glycol dicaprylate, propylene glycol di(caprylate caprate), propylene glycol dicaprate, propylene glycol dioleate, neopentyl glycol dicaprate, neopentyl glycol dioctanoate, glyceryl tricaprylate, glyceryl tri-2-ethylhexanoate, glyceryl tri(caprylate caprate), glyceryl tri(caprylate caprate stearate), glyceryl triundecylate, glyceryl triisopalmitate, glyceryl triisostearate, trimethylolpropane tri-2-ethylhexanoate, trimethylolpropane triisostearate, pentaerythrityl tetra-2-ethylhexanoate, pentaerythrityl tetramyristate, pentaerythrityl tetraisostearate, diglyceryl tetraisostearate, octyldodecyl neopentanoate, isocetyl octanoate, isostearyl octanoate, 2-ethylhexyl isopelargonate, hexyldecyl dimethyloctanoate, octyldodecyl dimethyloctanoate, 2-ethylhexyl isopalmitate, isocetyl isostearate, isostearyl isostearate, octyldodecyl isostearate, lauryl lactate, myristyl lactate, cetyl lactate, octyldodecyl lactate, triethyl citrate, acetyltriethyl citrate, acetyltributyl citrate, trioctyl citrate, triisocetyl citrate, trioctyldodecyl citrate, diisostearyl malate, 2-ethylhexyl hydroxystearate, di-2-ethylhexyl succinate, diisopropyl adipate, diisobutyl adipate, dioctyl adipate, diheptylundecyl adipate, diethyl sebacate, diisopropyl sebacate, dioctyl sebacate, cholesteryl stearate, cholesteryl isostearate, cholesteryl hydroxystearate, cholesteryl oleate, dihydrocholesteryl oleate, phytosteryl isostearate, phytosteryl oleate, isocetyl 12-stearoylhydroxystearate, stearyl 12-stearoylhydroxystearate, isostearyl 12-stearoylhydroxystearate, polyoxyethylene (3) polyoxypropylene (1) cetyl ether acetate, polyoxyethylene (3) polyoxypropylene (1) isocetyl ether acetate, isononyl isononanoate, octyl isononanoate, tridecyl isononanoate and isotridecyl isononanoate;
silicone oils such as methyl polysiloxane, methylphenyl polysiloxane, methylhydrogen polysiloxane, methyl cyclopolysiloxane, octamethyl cyclotetrasiloxane, decamethyl cyclopentasiloxane, dodecamethyl cyclohexasiloxane, octamethyl trisiloxane, decamethyl tetrasiloxane, tetradecamethyl hexasiloxane, highly polymerized methyl polysiloxane, dimethyl siloxane/methyl(polyoxyethylene)siloxane/methyl(polyoxypropylene)siloxane copolymer, dimethyl siloxane/methyl(polyoxyethylene)siloxane copolymer, dimethyl siloxane/methyl(polyoxypropylene)siloxane copolymer, dimethyl siloxane/methyl cetyloxysiloxane copolymer, dimethyl siloxane/methyl stearoxysiloxane copolymer, polyether-modified silicones, alcohol-modified silicones, alkyl-modified silicones and amino-modified silicones;
polyhydric alcohols such as ethylene glycol, diethylene glycol, triethylene glycol, polyethylene glycol, propylene glycol, dipropylene glycol, polypropylene glycol, pentanediol, glycerol, diglycerol, polyglycerol, isoprene glycol, 1,3-butylene glycol, 3-methyl-1,3-butanediol, 1,3-butanediol, 1,2-pentanediol and 1,2-hexanediol;
saccharides such as mannitol, sorbitol, xylitol, maltitol, erythritol, pentaerythritol, glucose, sucrose, fructose, lactose, maltose, xylose and trehalose;
polymers such as sodium alginate, carrageenan, agar, furcelleran, cyamoposis gum, pyrus cydonia seed, konjac mannan, tamarind gum, tara gum, dextrin, starch, ceratonia siliqua gum, gum arabic, ghatti gum, karaya gum, tragacanth gum, arabinogalactan, pectin, marmelo, chitosan, starch, curdlan, xanthan gum, gellan gum, cyclodextrin, dextran, pullulan, microcrystalline cellulose, methylcellulose, ethylcellulose, hydroxyethylcellulose, hydroxypropylcellulose, hydroxypropylmethylcellulose, carboxymethylcellulose, carboxy starch, cationized cellulose, starch phosphate, cationized cyamoposis gum, carboxymethyl/hydroxypropylated cyamoposis gum, hydroxypropylated cyamoposis gum, albumin, casein, gelatin, sodium polyacrylate, polyacrylic acid amide, carboxyvinyl polymers, polyethyleneimine, highly polymerized polyethylene glycol, polyvinyl alcohol, polyvinylpyrrolidone, polyvinyl ether, polyacrylamide, acrylic acid copolymers, methacrylic acid copolymers, maleic acid copolymers, vinylpyridine copolymers, ethylene/acrylic acid copolymers, vinylpyrrolidone polymers, vinyl alcohol/vinylpyrrolidone copolymers, nitrogen-substituted acrylamide polymers, amino-modified silicones, cationized polymers, dimethylacryl ammonium polymers, acrylic acid-based anionic polymers, methacrylic acid-based anionic polymers, modified silicones, alkyl (C₁₀₋₃₀) acrylate or methacrylate copolymers and polyoxyethylene/polyoxypropylene copolymer;
alcohols such as ethanol, isopropyl alcohol, 1-butanol, 2-butanol and benzyl alcohol;
anionic surfactants such as coconut fatty acid potassium, coconut fatty acid sodium, coconut fatty acid triethanolamine, potassium laurate, sodium laurate, triethanolamine laurate, potassium myristate, sodium myristate, isopropanolamine myristate, potassium palmitate, sodium palmitate, isopropanolamine palmitate, potassium stearate, sodium stearate, triethanolamine stearate, potassium oleate, sodium oleate, castor oil fatty acid sodium, zinc undecylenate, zinc laurate, zinc myristate, magnesium myristate, zinc palmitate, zinc stearate, calcium stearate, magnesium stearate, aluminum stearate, calcium myristate, magnesium myristate, aluminum dimyristate, aluminum isostearate, polyoxyethylene laurylether acetic acid, sodium polyoxyethylene laurylether acetate, polyoxyethylene tridecylether acetic acid, sodium polyoxyethylene tridecylether acetate, sodium stearoyl lactate, sodium isostearoyl lactate, sodium lauroylsarcosine, coconut fatty acid sarcosine, sodium coconut fatty acid sarcosine, coconut fatty acid sarcosine triethanolamine, lauroyl sarcosine, potassium lauroyl sarcosine, lauroyl sarcosine triethanolamine, oleyl sarcosine, sodium myristoyl sarcosine, sodium stearoyl glutamate, coconut fatty acid acylglutamic acid, potassium coconut fatty acid acylglutamate, sodium coconut fatty acid acylglutamate, coconut fatty acid acylglutamate triethanolamine, lauroyl acylglutamic acid, potassium lauroyl acylglutamate, sodium lauroyl acylglutamate, lauroyl acylglutamate triethanolamine, myristoyl acylglutamic acid, potassium myristoyl acylglutamate, sodium myristoyl acylglutamate, stearoyl acylglutamic acid, potassium stearoyl acylglutamate, disodium stearoyl acylglutamate, sodium hardened tallow fatty acid acylglutamate, sodium coconut fatty acid/hardened tallow fatty acid acylglutamate, sodium coconut fatty acid methylalanine, lauroyl methylalanine, sodium lauroyl methylalanine, lauroyl methylalanine triethanolamine, sodium myristoyl methylalanine, sodium lauroylmethyl taurine, potassium coconut fatty acid methyltaurine, sodium coconut fatty acid methyltaurine, magnesium coconut fatty acid methyltaurine, sodium myristoyl methyltaurine, sodium palmitoyl methyltaurine, sodium stearoyl methyltaurine, sodium oleoyl methyltaurine, sodium alkanesulfonate, sodium tetradecenesulfonate, dioctylsodium sulfosuccinate, disodium lauryl sulfosuccinate, sodium coconut fatty acid ethyl ester sulfonate, sodium laurylsulfate, triethanolamine laurylsulfate, sodium cetyl sulfate, triethanolamine alkylsulfates (11,13,15), sodium alkylsulfates (12,13), triethanolamine alkylsulfates (12,13), ammonium alkylsulfates (12,14,16), diethanolamine alkylsulfates (12,13), triethanolamine alkylsulfates (12-14), triethanolamine alkylsulfates (12-15), magnesium triethanolamine cocoalkylsulfate, ammonium laurylsulfate, potassium laurylsulfate, magnesium laurylsulfate, monoethanolamine laurylsulfate, diethanolamine laurylsulfate, sodium myristylsulfate, sodium stearylsulfate, sodium oleylsulfate, triethanolamine oleylsulfate, sodium polyoxyethylene laurylether sulfate, triethanolamine polyoxyethylene laurylether sulfate, sodium polyoxyethylene (1) alkyl (11,13,15)ether sulfate, triethanolamine polyoxyethylene (1) alkyl (11,13,15) ether sulfate, sodium polyoxyethylene (3) alkyl (11-15) ether sulfate, sodium polyoxyethylene (2) alkyl (12, 13) ether sulfate, sodium polyoxyethylene (3) alkyl (12-14) ether sulfate, sodium polyoxyethylene (3) alkyl (12-15) ether sulfate, sodium polyoxyethylene (2) laurylether sulfate, sodium polyoxyethylene (3) myristylether sulfate, sodium higher fatty acid alkanolamide sulfate, laurylphosphoric acid, sodium laurylphosphate, potassium cetylphosphate, diethanolamine cetylphosphate, polyoxyethylene oleylether phosphoric acid, polyoxyethylene laurylether phosphoric acid, sodium polyoxyethylene laurylether phosphate, polyoxyethylene cetylether phosphoric acid, sodium polyoxyethylene cetylether phosphate, polyoxyethylene stearylether phosphoric acid, polyoxyethylene oleylether phosphoric acid, sodium polyoxyethylene oleylether phosphate, polyoxyethylene alkylphenyl ether phosphoric acid, sodium polyoxyethylene alkylphenyl ether phosphate, triethanolamine polyoxyethylene alkylphenyl ether phosphate, polyoxyethylene octylether phosphoric acid, polyoxyethylene (10) alkyl (12,13) ether phosphoric acid, polyoxyethylene alkyl(12-15) ether phosphoric acid, polyoxyethylene alkyl (12-16) ether phosphoric acid, triethanolamine polyoxyethylene laurylether phosphate and diethanolamine polyoxyethylene oleylether phosphate;
cationic surfactants such as dioctylamine, dimethylstearylamine, trilaurylamine, stearic acid diethylaminoethylamide, lauryltrimethylammonium chloride, cetyltrimethylammonium chloride, cetyltrimethylammonium bromide, cetyltrimethylammonium saccharin, stearyltrimethylammonium chloride, alkyl (20-22) trimethylammonium chloride, lauryltrimethylammonium bromide, alkyl (16,18) trimethylammonium chloride, stearyltrimethylammonium bromide, stearyltrimethylammonium saccharin, alkyl (28) trimethylammonium chloride, di(polyoxyethylene)oleylmethylammonium chloride (2EO), dipolyoxyethylenestearylmethylammonium chloride, polyoxyethylene (1) polyoxypropylene (25) diethylmethylammonium chloride, tri(polyoxyethylene)stearylammonium chloride (5EO), distearyldimethylammonium chloride, dialkyl (12-15) dimethylammonium chloride, dialkyl (12-18) dimethylammonium chloride, dialkyl (14-18) dimethylammonium chloride, dicocoyldimethylammonium chloride, dicetyldimethylammonium chloride, isostearyllauryldimethylammonium chloride, benzalkonium chloride, myristyldimethylbenzylammonium chloride, lauryldimethyl(ethylbenzyl)ammonium chloride, stearyldimethylbenzylammonium chloride, laurylpyridinium chloride, cetylpyridinium chloride, lauroylcolaminoformylmethylpyridinium chloride, stearoylcolaminoformylmethylpyridinium chloride, alkylisoquinolium bromide, methylbenzethonium chloride and benzethonium chloride;
amphoteric surfactants such as 2-alkyl-N-carboxymethyl-N-hydroxyethyl imidazolinium betaine, alkyldiaminoethylglycine hydrochloride, sodium lauryldiaminoethylglycine, sodium undecylhydroxyethylimidazolium betaine, undecyl-N-carboxymethylimidazolium betaine, disodium coconut fatty acid acyl-N-carboxyethyl-N-hydroxyethylethylenediamine, disodium coconut fatty acid acyl-N-carboxyethoxyethyl-N-carboxyethylethylenediamine, disodium coconut fatty acid acyl-N-carboxymethoxyethyl-N-carboxymethylethylenediamine, sodium laurylaminopropionate, sodium laurylaminodipropionate, triethanolamine laurylaminopropionate, sodium palm oil fatty acid acyl-N-carboxyethyl-N-hydroxyethylethylenediamine, betaine lauryldimethylaminoacetate, betaine coconut oil alkyldimethylaminoacetic acid, betaine stearyldimethylaminoacetate, sodium stearyldimethyl betaine, coconut fatty acid amidopropylbetaine, palm oil fatty acid amidopropylbetaine, lauric acid amide betaine propylacetate, amidopropylbetaine ricinoleate, stearyldihydroxyethyl betaine and laurylhydroxysulfobetaine;
nonionic surfactants such as polyoxyethylene (10) alkyl (12,13) ether, polyoxyethylene lauryl ether, polyoxyethylene cetyl ether, polyoxyethylene stearyl ether, polyoxyethylene oleyl ether, polyoxyethylene (3,7,12) alkyl (12-14) ether, polyoxyethylene tridecyl ether, polyoxyethylene myristyl ether, polyoxyethylene-sec-alkyl (14) ether, polyoxyethylene isocetyl ether, polyoxyethylene cetostearyl ether, Polyoxyethylene (2,10,20) isostearyl ether, polyoxyethylene oleylcetyl ether, polyoxyethylene (20) aralkyl ether, polyoxyethylene octyldodecyl ether, polyoxyethylene behenyl ether, polyoxyethylene octylphenyl ether, polyoxyethylene nonylphenyl ether, polyoxyethylene dinonylphenyl ether, polyoxyethylene (1) polyoxypropylene (1,2,4,8) cetyl ether, polyoxyethylene (5) polyoxypropylene (1,2,4,8) cetyl ether, polyoxyethylene (10) polyoxypropylene (1,2,4,8) cetyl ether, polyoxyethylene (20) polyoxypropylene (1,2,4,8) cetyl ether, polyoxyethylene polyoxypropylene lauryl ether, polyoxyethylene (3) polyoxypropylene (34) stearyl ether, polyoxyethylene (4) polyoxypropylene (30) stearyl ether, polyoxyethylene (34) polyoxypropylene (23) stearyl ether, polyoxyethylene polyoxypropylene cetyl ether, polyoxyethylene polyoxypropylene decyltetradecyl ether, polyethylene glycol monolaurate, ethylene glycol monostearate, polyethylene glycol monostearate, polyethylene glycol monooleate, ethylene glycol fatty acid ester, self-emulsifiable ethylene glycol monostearate, diethylene glycol laurate, polyethylene glycol myristate, polyethylene glycol palmitate, diethylene glycol stearate, self-emulsifiable polyethylene glycol (2) monostearate, polyethylene glycol isostearate, ethylene glycol dioctanoate, diethylene glycol dilaurate, polyethylene glycol dilaurate, polyethylene glycol (150) dipalmitate, ethylene glycol distearate, diethylene glycol distearate, polyethylene glycol distearate, ethylene glycol dioleate, polyethylene glycol dioleate, polyethylene glycol diricinoleate, polyoxyethylene (20) sorbitan monolaurate, polyoxyethylene (20) sorbitan monopalmitate, polyoxyethylene (6) sorbitan monostearate, polyoxyethylene (20) sorbitan monostearate, polyoxyethylene (20) sorbitan tristearate, polyoxyethylene (6) sorbitan monooleate, polyoxyethylene (20) sorbitan monooleate, polyoxyethylene (20) sorbitan trioleate, polyoxyethylene (20) coconut fatty acid sorbitan, polyoxyethylene (10-80) sorbitan monolaurate, polyoxyethylene sorbitan tristearate, polyoxyethylene (20) sorbitan isostearate, polyoxyethylene (150) sorbitan tristearate, polyoxyethylene castor oil, polyoxyethylene hardened castor oil, polyoxyethylene (10) hardened castor oil, polyoxyethylene (20) hardened castor oil, polyoxyethylene (40) hardened castor oil, polyoxyethylene (50) hardened castor oil, polyoxyethylene (60) hardened castor oil, lipophilic glyceryl monostearate, lipophilic glyceryl monooleate, self-emulsifiable glyceryl monostearate, coconut fatty acid glyceryl, glyceryl laurate, glyceryl myristate, glyceryl isostearate, glyceryl ricinoleate, glyceryl monohydroxystearate, glyceryl oleate, glyceryl linoleate, glyceryl erucate, glyceryl behenate, wheat germ oil fatty acid glyceride, safflower oil fatty acid glyceryl, hydrogenated soybean fatty acid glyceryl, saturated fatty acid glyceride, cotton seed oil fatty acid glyceryl, monoisostearic acid glyceryl monomyristate, monotallow fatty acid glyceride, monoglyceryl lanolin fatty acid, glyceryl sesquioleate, glyceryl distearate, glyceryl diisostearate, glyceryl diarachidate, sorbitan monolaurate, sorbitan monopalmitate, sorbitan monostearate, sorbitan monoisostearate, sorbitan monooleate, sorbitan sesquistearate, sorbitan sesquioleate, sorbitan tristearate, sorbitan trioleate, coconut fatty acid sorbitan, sorbitan isostearate, sorbitan sesquiisostearate, sorbitan distearate, diglyceryl isopalmitate, poly (4-10) glyceryl monolaurate, poly (10) glyceryl monomyristate, poly (2-10) glyceryl monostearate, poly (2-10) glyceryl monoisostearate, poly (2-10) glyceryl monooleate, diglyceryl sesquioleate, poly (2-10) glyceryl diisostearate, poly (6-10) glyceryl distearate, diglyceryl triisostearate, poly (10) glyceryl tristearate, poly (10) glyceryl trioleate, poly (2) glyceryl tetraisostearate, decaglyceryl pentastearate, poly (6-10) glyceryl pentaoleate, poly (10) glyceryl heptastearate, decaglyceryl decastearate, poly (10) glyceryl decaoleate, condensed poly (6) glyceryl ricinoleate, sucrose fatty acid ester, sucrose coconut fatty acid ester, alkyl glucoside, coconut oil alkyldimethylamine oxide, lauryldimethylamine oxide, dihydroxyethyllauryldimethylamine oxide, stearyldimethylamine oxide, oleyldimethylamine oxide and polyoxyethylene coconut oil alkyldimethylamine oxide;
natural surfactants such as saponin, lecithin, soybean phospholipid, hydrogenated soybean phospholipid, soybean lysophospholipid, hydrogenated soybean lysophospholipid, egg yolk lecithin, hydrogenated egg yolk lysophosphatidylcholine, phosphatidylcholine, phosphatidylethanolamine, phosphatidylserine, sphingophospholipid, sphingomyelin, ganglioside, bile acid, cholic acid, deoxycholic acid, sodium cholate, sodium deoxycholate, spiculisporic acid, rhamnolipid, trehalose lipid, sophorolipid and mannosylerythritol lipid;
ultraviolet light absorbers, including paraaminobenzoic acid derivatives such as para-aminobenzoic acid, ethyl para-aminobenzoate, glyceryl para-aminobenzoate, amyl para-dimethylaminobenzoate and 2-ethylhexyl para-dimethylaminobenzoate, cinnamic acid derivatives such as benzyl cinnamate, dipara-methoxy cinnamic acid glyceryl mono-2-ethylhexanoate, methyl 2,4-diisopropylcinnamate, ethyl 2,4-diisopropylcinnamate, potassium para-methoxycinnamate, sodium para-methoxycinnamate, isopropyl para-methoxycinnamate, 2-ethylhexyl para-methoxycinnamate, 2-ethoxyethyl para-methoxycinnamate and ethyl para-ethoxycinnamate, urocanic acid derivatives such as urocanic acid and ethyl urocanate, benzophenone derivatives such as 2,4-dihydroxybenzophenone, 2,2',4,4'-tetrahydroxybenzophenone, 2-hydroxy-4-methoxy-5-sulfobenzophenone sodium, 2-hydroxy-4-methoxybenzophenone-5-sulfonic acid, 2-hydroxy-4-methoxybenzophenone, 2,2'-dihydroxy-4,4'-dimethoxybenzophenone and 2,2'-dihydroxy-4,4'-dimethoxy-5-sulfobenzophenone sodium, salicylic acid derivatives such as ethylene glycol salicylate, 2-ethylhexyl salicylate, phenyl salicylate, benzyl salicylate, p-tert-butylphenyl salicylate, homomenthyl salicylate and 3,3,5-trimethylcyclohexyl salicylate, 2-(2'-hydroxy-5'-methoxyphenyl)benzotriazole and 4-tert-butyl-4'-methoxybenzoylmethane;
powders and color materials, including natural dyes such as kaolin, silicic anhydride, aluminum magnesium silicate, sericite, talc, boron nitride, mica, montmorillonite, hemp cellulose powder, wheat starch, silk powder, cornstarch, nitro dye, azo dye, nitroso dye, triphenylmethane dye, xanthene dye, quinoline dye, anthraquinone dye, indigo dye, pyrene dye, phthalocyanine dye, flavonoid, quinone, porphyrin, water-soluble annatto, squid ink powder, caramel, guaiazulene, gardenia blue, gardenia yellow, cochineal, shikonin, sodium copper chlorophyllin, paprika dye, safflower red, safflower yellow, laccaic acid and riboflavin butyrate, carbon black, yellow iron oxide, black iron oxide, red iron oxide, iron blue, ultramarine blue, zinc oxide, chromium oxide, titanium oxide, black titanium oxide, zirconium oxide, chromium hydroxide, alumina, magnesium oxide, barium sulfate, aluminum hydroxide, calcium carbonate, lithium cobalt titanate, manganese violet and pearl pigment;
plant extracts such as angelica keiskei extract, gambir extract, avocado extract, hydrangea serrata leaf extract, gynostemma pentaphyllum extract, althea extract, arnica extract, oil-soluble arnica extract, almond extract, aloe extract, styrax benzoin resin extract, ginkgo extract, urtica extract, orris root extract, fennel extract, curcuma extract, rose fruit extract, echinacea leaf extract, scutellaria baicalensis root extract, phellodendron bark extract, coptis rhizome extract, hordeum vulgare seed extract, gumbo extract, hypericum erectum extract, oil-soluble hypericum erectum extract, lamium album flower extract, oil-soluble lamium album flower extract, ononis extract, nasturtium officinale extract, orange extract, orange flower water, seaweed extract, kaki tannin, puerariae radix extract, valerian extract, cattail extract, chamomilla extract, oil-soluble chamomilla extract, chamomilla water, oat extract, carrot extract, oil-soluble carrot extract, carrot oil, artemisia capillaris extract, licorice extract, licorice extract powder, licorice flavonoid, cantharis tincture, raspberry extract, kiwi extract, cinchona bark extract, cucumber extract, apricot kernel extract, quince seed extract, gardenia extract, sasa veitchii extract, sophora angustifolia extract, walnut shell extract, grapefruit extract, clematis extract, brown sugar extract, chlorella extract, mulberry extract, cinnamon bark extract, gentian extract, geranium herb extract, tea extract, spatterdock extract, arctium lappa root extract, oil-soluble arctium lappa root extract, wheat germ extract, hydrolyzed wheat powder, rice bran extract, rice bran fermentation extract, comfrey extract, asiasarum root extract, saffron extract, saponaria officinalis extract, oil-soluble salvia extract, crataegus cuneata fruit extract, xanthoxylum extract, shiitake mushroom extract, shiitake mushroom extract powder, rehmannia glutinosa extract, sycon extract, oil-soluble sycon extract, Japanese basil extract, linden extract, oil-soluble linden extract, filipendula multijuga extract, peony root extract, coix lacryma-jobi seed extract, ginger extract, oil-soluble ginger extract, ginger tincture, acorus calamus root extract, betula alba extract, oil-soluble betula alba extract, betula alba sap, lonicera extract, equisetum arvense extract, oil-soluble equisetum arvense extract, scordinin, stevia extract, ivy extract, crataegus oxyacantha extract, sambucus nigra flower extract, juniperus communis extract, achillea millefolium extract, oil-soluble achillea millefolium extract, mentha piperita extract, sage extract, oil-soluble sage extract, sage water, mallow extract, celery extract, cnidium officinale extract, cnidium officinale water, swertia japonica extract, soybean extract, jujube extract, thyme extract, camellia sinensis leaf extract, camellia sinensis dry distillate, camellia sinensis seed extract, clove flower extract, citrus unshiu peel extract, camellia japonica seed extract, centella asiatica extract, oil-soluble juglans regia extract, duke extract, terminalia extract, capsicum tincture, angelica acutiloba extract, oil-soluble angelica acutiloba extract, angelica acutiloba water, calendula officinalis flower extract, oil-soluble calendula officinalis flower extract, soymilk powder, prunus persica extract, citrus aurantium amara extract, houttuynia cordata extract, tomato extract, potentilla erecta root extract, natto extract, ginseng extract, oil-soluble ginseng extract, garlic extract, rosa canina fruit extract, oil-soluble rosa canina fruit extract, malt extract, malt root extract, ophiopogon tuber extract, parsley extract, hordeum vulgare leaf juice concentrate, distilled peppermint water, hamamelis water, hamamelis extract, rosa centifolia flower extract, parietaria extract, isodonis japonicus extract, eriobotrya japonica leaf extract, oil-soluble eriobotrya japonica leaf extract, coltsfoot flower extract, poria cocos extract, ruscus aculeatus root extract, ruscus aculeatus root extract powder, grape extract, grape leaf extract, grape water, hayflower extract, luffa cylindrica fruit extract, luffa cylindrica fruit water, safflower extract, oil-soluble tilia platyphyllos flower extract, tilia platyphyllos flower water, paeonia suffruticosa root extract, hops extract, oil-soluble hops extract, pinus sylvestris cone extract, silybum marianum fruit extract, horse chestnut extract, oil-soluble horse chestnut extract, sapindus mukurossi peel extract, melissa officinalis leaf extract, melilotus officinalis extract, peach leaf extract, oil-soluble peach leaf extract, bean-sprouts extract, centaurea cyanus flower extract, centaurea cyanus flower water, eucalyptus extract, saxifraga sarmentosa extract, lilium candidum bulb extract, coix lacryma jobi seed extract, oil-soluble coix lacryma jobi seed extract, artemisia princeps extract, artemisia princeps water, lavender extract, lavender water, apple extract, ganoderma lucidum extract, lettuce extract, lemon extract, astragalus sinicus extract, rose water, rosemary extract, oil-soluble rosemary extract, anthemis nobilis flower extract and sanguisorba officinalis root extract;
amino acids and peptides such as glycine, alanine, valine, leucine, isoleucine, serine, threonine, phenylalanine, thyrosin, tryptophan, cystine, cysteine, methionine, proline, hydroxyproline, aspartic acid, asparagine, glutamic acid, glutamine, arginine, histidine, lysine, γ-aminobutyric acid, DL-pyrrolidonecarboxylic acid, ε-aminocaproic acid, hydrolyzed elastin, water-soluble elastin, hydrolyzed collagen, water-soluble collagen, casein, glutathione, wheat peptide and soybean peptide;
vitamins and vitamin affecters, including vitamin A such as retinol, retinal, retinoic acid, retinol acetate and retinol palmitate, carotenoids such as α-carotene, β-carotene, γ-carotene, δ-carotene, lycopene, zeaxanthin, cryptoxanthin, echinenone and astaxanthin, vitamin B1 such as thiamines, vitamin B2 such as riboflavin, vitamin B6 such as pyridoxine, pyridoxal and pyridoxamine, vitamin B12 such as cyanocobalamin, vitamin C such as folic acids, nicotinic acid, nicotinic acid amide, pantothenic acids, biotins, L-ascorbic acid, sodium L-ascorbate, L-ascorbyl stearate, L-ascorbyl palmitate, L-ascorbyl dipalmitate, L-ascorbyl tetraisopalmitate, disodium L-ascorbate sulfate, L-ascorbyl magnesium, L-ascorbyl sodium phosphate, 2-glucoside L-ascorbate and L-ascorbic acid magnesium 2-phosphate, vitamin D such as ergocalciferol and cholecalciferol, vitamin E such as d-α-tocopherol, DL-α-tocopherol, dl-α-tocopherol acetate, dl-α-tocopherol succinate, β-tocopherol, γ-tocopherol and d-δ-tocopherol, ubiquinones, vitamin K, carnitine, ferulic acid, γ-oryzanol, α-lipoic acid and orotic acid;
antiseptics such as benzoic acid, sodium benzoate, undecylenic acid, salicylic acid, sorbic acid, potassium sorbate, dehydroacetic acid, sodium dehydroacetate, isobutyl para-oxybenzoate, isopropyl para-oxybenzoate, ethyl para-oxybenzoate, butyl para-oxybenzoate, propyl para-oxybenzoate, benzyl para-oxybenzoate, methyl para-oxybenzoate, methyl sodium para-oxybenzoate, phenoxyethanol, photosensitive agent No. 101, photosensitive agent No. 201 and photosensitive agent No. 401;
antioxidants such as butylhydroxyanisole, butylhydroxytoluene, propyl gallate, erythorbic acid, sodium erythorbate, para-hydroxyanisole and octyl gallate;
sequestering agents such as trisodium ethylenediaminehydroxyethyltriacetate, edetic acid, disodium edetate, trisodium edetate, tetrasodium edetate, sodium citrate, gluconic acid, phytic acid, sodium polyphosphate and sodium metaphosphate;
moisturizers such as hyaluronic acid, sodium hyaluronate, sodium chondroitinsulfate, sodium lactate, sodium pyrrolidonecarboxylate, betaine, lactic acid bacteria culture solution, yeast extract and ceramide;
antiinflammatory agents such as glycyrrhizinic acid, trisodium glycyrrhizinate, dipotassium glycyrrhizinate, monoammonium glycyrrhizinate, β-glycyrrhetinic acid, glycerol glycyrrhetinate, stearyl glycyrrhetinate, lysozyme chloride, hydrocortisone and allantoin;
pH adjusters such as sodium hydroxide, potassium hydroxide and triethanolamine;
salts such as sodium chloride, potassium chloride, magnesium chloride and sodium sulfate;
α-hydroxy acids such as citric acid, glycolic acid, tartaric acid and lactic acid;
whitening agents such as arbutin, α-arbutin and placental extract;
essential oils such as angelica oil, ylang ylang oil, elemi oil, orange oil, German chamomile oil, anthemis nobilis oil, cardamom oil, calamus oil, galbanum oil, camphor oil, carrot seed oil, clary sage oil, grapefruit oil, clove oil, cinnamon bark oil, coriander oil, cypress oil, sandalwood oil, cedarwood oil, citronella oil, cinnamon leaf oil, jasmine absolute, juniper berry oil, ginger extract, spearmint oil, sage oil, cedar oil, geranium oil, thyme oil, tea tree oil, nutmeg oil, niaouli oil, neroli oil, pine oil, basil oil, peppermint oil, patchouli oil, palmarosa oil, fennel oil, petitgrain oil, black pepper oil, frankincense oil, vetivert oil, peppermint oil, bergamot oil, benzoin oil, aniba rosaeodora oil, marjoram oil, mandarin oil, myrrh oil, melissa oil, eucalyptus oil, Chinese lemon oil, lime oil, ravensara oil, lavandin oil, lavender oil, lindane oil, lemon oil, lemongrass oil, rose oil, rosewood oil, rosemary oil and lovage oil;
terpenes such as limonene, pinene, terpinene, terpinolene, myrcene and longifolene;
perfumes and water.

The external preparation for skin according to the present invention may further contain existing cosmetic ingredients at commonly used concentrations. For example, any of the cosmetic ingredients listed in the following documents are employable: The Japanese Standards of Cosmetic Ingredients 2nd edition (edited by Society of Japanese Pharmacopoeia and published by Yakuji Nippo, Ltd. (1984)), The Japanese Cosmetic Ingredients Codex (edited by Ministry of Health and Welfare, Pharmaceutical Examination Division and published by Yakuji Nippo, Ltd. (1993)), Supplement to The Japanese Cosmetic Ingredients Codex (edited by Ministry of Health and Welfare, Pharmaceutical Examination Division and published by Yakuji Nippo, Ltd. (1993)), The Comprehensive Licensing Standards of Cosmetics by Category (edited by Ministry of Health and Welfare, Pharmaceutical Examination Division and published by Yakuji Nippo, Ltd. (1993)), The Japanese Cosmetic Ingredients Codex by Category (edited by Ministry of Health and Welfare, Pharmaceutical Examination Division and published by Yakuji Nippo, Ltd. (1997)), and Dictionary of Cosmetic Ingredients (Nikko Chemicals., Co. Ltd. (1991)).

### [Effect of the invention]

The saccharide-combined inositol derivative compounded in a skin external preparation enables the external preparation to be spread over the skin without grain feeling and to provide excellent effects of giving a sufficient moist feeling and keeping the skin healthy.

### EXAMPLES

Hereinbelow, the present invention will be described in greater detail by Examples. However, it should be construed that the invention is not limited thereto. The percentage "%" refers to "% by mass" unless otherwise mentioned.

### [Example 1]

1 g of myo-inositol and 1 g of β-cyclodextrin were sufficiently dissolved in 20 ml of 50 mM sodium citrate buffer solution (pH 5.5). 500U of cyclodextrin glucanotransferase was added to the solution, and reaction was performed at 55°C for 48 hours. The reaction product was analyzed by LC-MS. The analysis confirmed that β-cyclodextrin disappeared in the reaction liquid obtained by the 48-hour reaction. After the reaction, the reaction liquid was boiled for 10 minutes to deactivate the enzyme. Subsequently, the reaction liquid was sufficiently cooled, filtered, frozen and freeze-dried. Thus, 1.2 g of white powder containing glucosyl inositol (hereinafter abbreviated to as GI-1) was obtained. LC-MS analysis of the GI-1 provided that of the glucose chains combined to the inositol, 38% had one glucose, 28% had two glucoses, 10% had three glucoses, 4% had four glucoses and 1% had five glucoses. The GI-1 was odorless and had very high solubility in water.

### [Example 2]

A lotion as shown in Table 1 was prepared by a common method (Sample 1-1). Ten female volunteers between the ages of 20 and 30 were asked to use the lotion and their impressions were obtained. The result is given in Table 2. The result shows that the sample 1-1 containing the GI-1 caused no grain feeling, had no distinctive odor, and improved moist feeling of the skin.

### [Comparative Examples 1 and 2]

Lotions as shown in Table 1 were prepared by a common method (Samples 1-2 and 1-3). The samples were tested as described in Example 2 and after-use impressions were obtained. The results are given in Table 2.

**[Table 1]**

| | Example 2 | Comparative Example 1 | Comparative Example 2 |
|---|---|---|---|
| Sample | 1-1 | 1-2 | 1-3 |
| GI-1 | 1.00% | - | - |
| Rice bran extract | - | 1.0% | - |
| 1,3-Butylene glycol | 5.00% | 5.00% | 5.00% |
| Ethyl alcohol | 5.00% | 5.00% | 5.00% |
| Citric acid | 0.01% | 0.01% | 0.01% |
| Sodium citrate | 1.00% | 1.00% | 1.00% |
| Methylparaben | 0.20% | 0.20% | 0.20% |
| Purified water | 87.79% | 87.79% | 88.79% |
| | 100.00% | 100.00% | 100.00% |

**[Table 2]**

| | Example 2 | Comparative Example 1 | Comparative Example 2 |
|---|---|---|---|
| Sample | 1-1 | 1-2 | 1-3 |
| Moist feeling after use | 9 | 4 | 2 |
| | | | |
| Grain feeling during use | 0 | 4 | 0 |
| | | | |
| No change of feeling | 1 | 2 | 8 |
| | | | |
| Distinctive odor | 0 | 8 | 0 |

### [Example 3]

A milky lotion as shown in Table 3 was prepared by a common method (Sample 2-1). Ten female volunteers between the ages of 20 and 30 were asked to use the milky lotion and their impressions were obtained. The result is given in Table 4. The result shows that the sample -1 containing the GI-1 caused no grain feeling, had no distinctive odor, and improved moist feeling of the skin.

### [Comparative Examples 3 and 4]

Milky lotions as shown in Table 3 were prepared by a common method (Samples 2-2 and 2-3). The samples were tested as described in Example 3 and after-use impressions were obtained. The results are given in Table 4.

**[Table 3]**

| | Example 3 | Comparative Example 3 | Comparative Example 4 |
|---|---|---|---|
| Sample | 2-1 | 2-2 | 2-3 |
| GI-1 | 1.00% | - | - |
| Rice bran extract | - | 1.0% | - |
| Glycerol | 8.00% | 8.00% | 8.00% |
| 1,3-Butylene glycol | 2.00% | 2.00% | 2.00% |
| Sodium hyaluronate | 0.05% | 0.05% | 0.05% |
| Hydroxyethylcellulose | 0.30% | 0.30% | 0.30% |
| Xanthan gum | 0.30% | 0.30% | 0.30% |
| Sodium citrate | 1.00% | 1.00% | 1.00% |
| Polyethylene glycol-50 | 0.50% | 0.50% | 0.50% |
| Methylparaben | 0.20% | 0.20% | 0.20% |
| Purified water | 86.65% | 86.65% | 87.65% |
| | 100.00% | 100.00% | 100.00% |

**[Table 4]**

| | Example 3 | Comparative Example 3 | Comparative Example 4 |
|---|---|---|---|
| Sample | 2-1 | 2-2 | 2-3 |
| Moist feeling after use | 8 | 5 | 4 |
| | | | |
| Grain feeling during use | 0 | 5 | 0 |
| | | | |
| No change of feeling | 2 | 2 | 6 |
| | | | |
| Distinctive odor | 0 | 7 | 0 |

### [Example 4]

10 g of myo-inositol and 10 g of β-cyclodextrin were sufficiently dissolved in 200 ml of 50 mM sodium citrate buffer solution (pH 5.5). 5000U of cyclodextrin glucanotransferase was added to the solution, and reaction was performed at 55°C for 48 hours. The reaction product was analyzed by LC-MS. The analysis confirmed that β-cyclodextrin disappeared in the reaction liquid obtained by the 48-hour reaction. After the reaction, the reaction liquid was boiled for 15 minutes to deactivate the enzyme. Subsequently, the reaction liquid was sufficiently cooled, filtered, frozen and freeze-dried. Thus, 13.7 g of white powder containing glucosyl inositol (hereinafter abbreviated to as GI-2) was obtained. LC-MS analysis of the GI-2 provided that of the glucose chains combined to the inositol, 42% had one glucose, 30% had two glucoses, 9% had three glucoses, 3% had four glucoses and 1% had five glucoses.

Thereafter, 12 g of the GI-2 was dissolved in 5 ml of purified water to give a sample solution for preparative liquid chromatography. Preparative liquid chromatography was carried out under conditions such that the column was Shodex SUGAR KS-2002, the guard column was Shodex SUGAR KS-LG, the eluting solution was purified water, the flow rate was 5 ml/min and the column temperature was 80°C. The peaks were monitored using detector RI, and the peaks assigned to the oligosaccharide combined to the inositol residue were selectively collected, and the fraction was recovered. The recovered liquid was frozen and freeze-dried. Thus, 8.5 g of white powder containing glucosyl inositol (hereinafter abbreviated to as GI-3) was obtained. LC-MS analysis of the GI-3 provided that of the glucose chains combined to the inositol, 51% had one glucose, 35% had two glucoses, 10% had three glucoses, 3% had four glucoses and 1% had five glucoses. The GI-3 was odorless and had very high solubility in water.

### [Example 5]

A lotion as shown in Table 5 was prepared by a common method (Sample 3-1). Three female volunteers between the ages of 30 and 40 were asked to apply the lotion on forearm flexor aspects over a period of 7 days, two times a day in the morning and evening. On the 8th morning, the ceratoid moisture content was examined by measuring the conductance by a high-frequency inductance method using an impedance meter (SKICON-200 manufactured by IBS). The result is given in Table 6. The result shows that the sample 3-1 containing the GI-3 achieved higher conductance and increased the ceratoid moisture content to improve the moist feeling.

### [Comparative Examples 5 to 7]

Lotions as shown in Table 5 were prepared by a common method (Samples 3-2 to 3-4). The samples were tested as described in Example 5. The results are given in Table 6.

**[Table 5]**

| | Ex. 5 | Comp. 5 Ex. | Comp. 6 Ex. | Comp. 7 Ex. |
|---|---|---|---|---|
| Sample | 3-1 | 3-2 | 3-3 | 3-4 |
| GI-3 | 3.0% | - | - | - |
| Inositol | - | 3.0% | - | - |
| Glucose | - | - | 3.0% | - |
| Butylene glycol | 9.5% | 9.5% | 9.5% | 9.5% |
| Ethyl alcohol | 39.6% | 39.6% | 39.6% | 39.6% |
| Glycine | 0.5% | 0.5% | 0.5% | 0.5% |
| Castor oil | 4.9% | 4.9% | 4.9% | 4.9% |
| Methylparaben | 0.2% | 0.2% | 0.2% | 0.2% |
| Purified water | 42.3% | 45.3% | 45.3% | 45.3% |
| | 100.0% | 100.0% | 100.0% | 100.0% |

**[Table 6]**

| | | Ex. 5 | Comp. Ex. 5 | Comp. Ex. 6 | Comp. Ex. 7 |
|---|---|---|---|---|---|
| | Before application | Sample 3-1 | Sample 3-2 | Sample 3-3 | Sample 3-4 |
| Conductance | 100 | 155 | 135 | 122 | 120 |

### [Example 6]

A milky lotion as shown in Table 7 was prepared by a common method (Sample 4-1). Similarly to Example 5, three female volunteers aged from 30 to 40 were asked to apply the milky lotion on forearm flexor aspects over a period of 7 days, two times a day in the morning and evening. On the 8th morning, the ceratoid moisture content was examined by measuring the conductance using an impedance meter. The result is given in Table 8. The result shows that the sample 4-1 containing the GI-3 achieved higher conductance and increased the ceratoid moisture content to improve the moist feeling.

### [Comparative Examples 8 to 10]

Milky lotions as shown in Table 7 were prepared by a common method (Samples 4-2 to 4-4). The samples were tested as described in Example 5. The results are given in Table 8.

**[Table 7]**

| | Ex. 6 | Comp. Ex. 8 | Comp. Ex. 9 | Comp. Ex. 10 |
|---|---|---|---|---|
| Sample | 4-1 | 4-2 | 4-3 | 4-4 |
| GI-3 | 3.0% | - | - | - |
| Inositol | - | 3.0% | - | - |
| Glucose | - | - | 3.0% | - |
| 1,3-Butylene glycol | 10.1% | 10.1% | 10.1% | 10.1% |
| Avocado oil | 11.0% | 11.0% | 11.0% | 11.0% |
| Behenyl alcohol | 0.6% | 0.6% | 0.6% | 0.6% |
| Stearic acid | 0.4% | 0.4% | 0.4% | 0.4% |
| Glycerol fatty acid ester Polyoxyethylene | 0.9% | 0.9% | 0.9% | 0.9% |
| Sorbitan fatty acid ester | 1.1% | 1.1% | 1.1% | 1.1% |
| Polyoxyethylene alkylether | 0.4% | 0.4% | 0.4% | 0.4% |
| Polyoxyethylene alkylether | 10.1% | 10.1% | 10.1% | 10.1% |
| Methylparaben | 0.2% | 0.2% | 0.2% | 0.2% |
| Purified water | 62.2% | 62.2% | 62.2% | 65.2% |
| | 100.0% | 100.0% | 100.0% | 100.0% |

**[Table 8]**

| | | Ex. 6 | Comp. Ex. 8 | Comp. Ex. 9 | Comp. Ex. 10 |
|---|---|---|---|---|---|
| | Before application | Sample 4-1 | Sample 4-2 | Sample 4-3 | Sample 4-4 |
| Conductance | 100 | 197 | 171 | 149 | 146 |

### [Example 7]

A cream as shown in Table 9 was prepared by a common method (Sample 5-1). Similarly to Example 5, three female volunteers between the ages of 30 and 40 were asked to apply the cream on forearm flexor aspects over a period of 7 days, two times a day in the morning and evening. On the 8th morning, the ceratoid moisture content was examined by measuring the conductance using an impedance meter. The result is given in Table 10. The result shows that the sample 5-1 containing the GI-3 achieved higher conductance and increased the ceratoid moisture content to improve the moist feeling.

### [Comparative Examples 11 to 13]

Creams as shown in Table 9 were prepared by a common method (Samples 5-2 to 5-4). The samples were tested as described in Example 5. The results are given in Table 10.

**[Table 9]**

| | Ex. 7 | Comp. Ex. 11 | Comp. Ex. 12 | Comp. Ex. 13 |
|---|---|---|---|---|
| Sample | 5-1 | 5-2 | 5-3 | 5-4 |
| GI-3 | 3.0% | - | - | - |
| Inositol | - | 3.0% | - | - |
| Glucose | - | - | 3.0% | - |
| 1,3-Butylene glycol | 11.9% | 11.9% | 11.9% | 11.9% |
| Squalane | 11.1% | 11.1% | 11.1% | 11.1% |
| Stearic acid | 7.8% | 7.8% | 7.8% | 7.8% |
| Stearyl alcohol | 6.0% | 6.0% | 6.0% | 6.0% |
| Beeswax | 1.9% | 1.9% | 1.9% | 1.9% |
| Propylene glycol monostearate | 3.1% | 3.1% | 3.1% | 3.1% |
| Polyoxyethylene cetyl ether | 1.1% | 1.1% | 1.1% | 1.1% |
| Methylparaben | 0.2% | 0.2% | 0.2% | 0.2% |
| Purified water | 53.9% | 53.9% | 53.9% | 56.9% |
| | 100.0% | 100.0% | 100.0% | 100.0% |

**[Table 10]**

| | | Ex. 7 | Comp. Ex. 11 | Comp. Ex. 12 | Comp. Ex. 13 |
|---|---|---|---|---|---|
| | Before application | Sample 5-1 | Sample 5-2 | Sample 5-3 | Sample 5-4 |
| Conductance | 100 | 237 | 222 | 191 | 190 |

## Claims

1. The use of a preparation comprising an inositol derivative which is a compound in which an inositol is combined with a saccharide, as a preparation for the external application to skin.

2. The use according to claim 1, wherein the saccharide is a monosaccharide and/or an oligosaccharide.

3. The use according to claim 2, wherein the monosaccharide and/or the oligosaccharide contains glucose as a constitutional unit.

4. The use according to any one of claims 1 to 3, wherein the inositol of the inositol derivative is myo-inositol.

5. The use according to any one of claims 1 to 4, wherein the inositol derivative
in which the inositol is combined with the saccharide constitutes 0.01 to 50% by mass of the preparation.

## Patentansprüche

1. Verwendung einer Zubereitung, die ein Inositolderivat enthält, das eine Verbindung ist, in der ein Inositol mit einem Saccharid kombiniert ist, als Zubereitung für die äußere Auftragung auf die Haut.

2. Verwendung nach Anspruch 1, wobei das Saccharid ein Monosaccharid und/oder ein Oligosaccharid ist.

3. Verwendung nach Anspruch 2, wobei das Monosaccharid und/oder das Oligosaccharid Glucose als Konstitutionseinheit enthält.

4. Verwendung nach einem der Ansprüche 1 bis 3, wobei das Inositol des Inositolderivats Myoinositol ist.

5. Verwendung nach einem der Ansprüche 1 bis 4, wobei das Inositolderivat, in dem das Inositol mit dem Saccharid kombiniert ist, 0,01 bis 50 Massen-% der Zubereitung ausmacht.

## Revendications

1. Utilisation d'une préparation comprenant un dérivé d'inositol qui est un composé dans lequel un inositol est combiné avec un saccharide comme préparation pour l'application externe sur la peau.

2. Utilisation selon la revendication 1, dans laquelle le saccharide est un monosaccharide et/ou un oligosaccharide.

3. Utilisation selon la revendication 2, dans laquelle le monosaccharide et/ou l'oligosaccharide contient du glucose comme unité constitutionnelle.

4. Utilisation selon l'une quelconque des revendications 1 à 3, dans laquelle l'inositol du dérivé d'inositol est le myo-inositol.

5. Utilisation selon l'une quelconque des revendications 1 à 4, dans laquelle le dérivé d'inositol dans lequel l'inositol est combiné avec le saccharide constitue de 0,01 à 50 % en masse de la préparation.
